# EUROPEAN PATENT SPECIFICATION

(11) **EP 2 773 367 B1**
(45) Date of publication and mention of the grant of the patent: **09.09.2015**
(21) Application number: 12780667.7
(22) Date of filing: 30.10.2012
(51) Int. Cl.: A61K 38/17, A61P 17/02

(54) **S100 FAMILY PROTEINS AND THEIR USES**
PROTEINE DER S-100-FAMILIE UND DEREN VERWENDUNGEN
PROTÉINES DE LA FAMILLE S100 ET LEURS UTILISATIONS

(30) Priority: 31.10.2011 EP 11008711
(43) Date of publication of application: 10.09.2014
(73) Proprietor: Ludwig-Maximilians-Universität München, 80539 München (DE)
(72) Inventor: GAUGLITZ, Gerd, 80469 München (DE); WOLF, Ronald, 80801 München (DE)
(74) Representative: Engelhard, Markus
(86) International application number: PCT/EP2012/004546
(87) International publication number: WO 2013/064244

(56) References cited:
- WO-A1-2008/070117
- WO-A1-2010/048716
- US-A1- 2010 055 694
- RONALD WOLF ET AL: "Novel S100A7 (psoriasin)/S100A15 (koebnerisin) subfamily: highly homologous but distinct in regulation and function", AMINO ACIDS ; THE FORUM FOR AMINO ACID AND PROTEIN RESEARCH, SPRINGER-VERLAG, VI, vol. 41, no. 4, 2 July 2010 (2010-07-02), pages 789-796, XP019951744, ISSN: 1438-2199, DOI: 10.1007/S00726-010-0666-4
- ZWICKER S ET AL: "Friend or Foe? - Psoriasin and Koebnerisin: multifunctional defence molecules in skin differentiation, tumorigenesis and inflammation", DEUTSCHE MEDIZINISCHE WOCHENSCHRIFT,, vol. 137, no. 10, 1 March 2012 (2012-03-01), pages 491-494, XP009157399, ISSN: 1439-4413
- WOLF RONALD ET AL: "S100A7 (psoriasin) and S100A15 (Koebnerisin) are almost identical in sequence but highly distinct in function: implications for disease pathogenesis", AMINO ACIDS, SPRINGER VERLAG, AU, vol. 37, no. Suppl. 1, 7 August 2009 (2009-08-07), page 78, XP009157407, ISSN: 0939-4451
- HU Z-F ET AL: "Differential expression of S100 protein gene in pathological scar", CHINESE JOURNAL OF CLINICAL REHABILITATION, CN, vol. 10, no. 32, 25 August 2006 (2006-08-25), pages 86-88, XP009157443, ISSN: 1671-5926

### SEQUENCE LISTING

<110> Ludwig-Maximilians-Universität Munchen
<120> S100 Family Proteins and Their Uses
<130> FB25168
<160> 2
<170> PatentIn version 3.5
<210> 1
   <211> 101
   <212> PRT
   <213> Homo sapiens
<400> 1
   Met Ser Asn Thr Gln Ala Glu Arg Ser Ile Ile Gly Met Ile Asp Met
   1               5                   10                  15
   Phe His Lys Tyr Thr Arg Arg Asp Asp Lys Ile Asp Lys Pro Ser Leu
               20                  25                  30
   Leu Thr Met Met Lys Glu Asn Phe Pro Asn Phe Leu Ser Ala Cys Asp
           35                  40                  45
   Lys Lys Gly Thr Asn Tyr Leu Ala Asp Val Phe Glu Lys Lys Asp Lys
       50                  55                  60
   Asn Glu Asp Lys Lys Ile Asp Phe Ser Glu Phe Leu Ser Leu Leu Gly
   65                  70                  75                  80
   Asp Ile Ala Thr Asp Tyr His Lys Gln Ser His Gly Ala Ala Pro Cys
                   85                  90                  95
   Ser Gly Gly Ser Gln
               100
<210> 2
   <211> 101
   <212> PRT
   <213> Homo sapiens
<400> 2
   Met Ser Asn Thr Gln Ala Glu Arg Ser Ile Ile Gly Met Ile Asp Met
   1               5                   10                  15
   Phe His Lys Tyr Thr Gly Arg Asp Gly Lys Ile Glu Lys Pro Ser Leu
               20                  25                  30
   Leu Thr Met Met Lys Glu Asn Phe Pro Asn Phe Leu Ser Ala Cys Asp
           35                  40                  45
   Lys Lys Gly Ile His Tyr Leu Ala Thr Val Phe Glu Lys Lys Asp Lys
       50                  55                  60
   Asn Glu Asp Lys Lys Ile Asp Phe Ser Glu Phe Leu Ser Leu Leu Gly
   65                  70                  75                  80
   Asp Ile Ala Ala Asp Tyr His Lys Gln Ser His Gly Ala Ala Pro Cys
                   85                  90                  95
   Ser Gly Gly Ser Gln
               100

## Description

### Field of the invention

The present invention relates to S100 family proteins and their uses. Moreover, the present invention relates to pharmaceutical compositions comprising an S100 family protein.

### Background of the invention

The skin is the largest organ of the human body. It fulfills important functions as a first line of defence against pathogens, as a sense organ, by retaining nutrients and water, and by heaping to regulate the body's temperature.

There are various kinds of skin disorders and diseases that are associated with an impaired state or function of the skin. This includes skin diseases caused by allergies and inflammation (such as allergic contact dermatitis, eczema, rosacea, or psoriasis), skin disorders caused by infections (such as abscess, shingles, or warts), and skin diseases resulting from various other causes (such as acne or sunburn).

Many of these skin disorders can cause serious pain and discomfort. In the modern-day society, the skin has become a sign of health and attractiveness, so skin disorders can greatly impact self-image and self-esteem. Some skin disorders may even be life-threatening, such as skin cancer.

Hypertrophic scars as well as keloids represent pathologically altered wound healing with excessive formation of scar tissue and reduced decomposition of dermal collagen [1,6]. Often, also an increased expression of proteins like fibronectin, laminin, and alpha smooth muscle actin is observed. Recently, several studies have shown that increased inflammation reaction and the subsequent release of different growth factors (such as TGF-β (transforming growth factor-beta)) by fibroblasts and keratinocytes leads to disturbed homeostasis within the connective tissue. An involvement of genetic factors in the formation of hypertrophic scars and keloids has been discussed because of an increased incidence of these skin disorders in certain families.

Based on current findings, a promising therapeutic approach of hypertrophic scars and keloids is questionable as the original defect has not been discovered. However, medications and therapies used today can reduce size, spread and volume of hypertrophic scars to a certain extent and achieve an improved cosmetic result and decrease distress, like itching, pain and the sensation of tension. However, most therapies are associated with considerable risks, a high probability of side effects, and high recurrence rates. Novel approaches for anti-proliferative treatment of hypertrophic scars are therefore a central area of research in dermatology.

Fibroproliferative disorders (FPDs) are common and frequently lethal disorders involving a diversity of human tissues, including cirrhosis and fibrosis of the liver and idiopathic pulmonary fibrosis [2]. Hypertrophic scars (HSc) and keloids represent the cutaneous equivalents of FPD. Both conditions are associated with significant morbidity by causing pruritus, pain, and/or contractures which consequently affect the patient's quality of life. HSc and keloids form during disturbed wound healing and evolve after injuries of the deep dermis. To date, the pathophysiology of hypertrophic scar and keloid formation is not well understood. Most therapeutic approaches remain unsatisfactory, due to poor knowledge of the complex mechanisms underlying the process of excessive scarring.

Excessive scar tissue formation results from increased deposition of large amounts of extracellular matrix (ECM) in the dermis. Excessive scars are densely populated by fibroblasts and inflammatory cells which maintain a fibrogenic micromilieu by secreting transforming growth factors-β (TGF-β) and other cytokines. This leads to increased production of collagen, proteoglycans, and fibronectin, deficient ECM degradation and remodelling [3]. Current research in keloid pathophysiology further suggests that epidermal-dermal interactions [4], alterations of the fibroblast phenotype through several growth factors and downstream signalling pathways [5] are involved in excessive scarring. In particular, TGF-β cytokines and downstream Smad pathways are key regulators in early stages of normal and hypertrophic scar formation. While TGF-β1 and -2 represent the most important stimulators of collagen and proteoglycan synthesis [6,7], administration of TGF-β3 reduces connective tissue deposition [8]. Despite encouraging results using recombinant human TGF-β3 or anti-TGF-β1 and -2 to prevent exaggerated collagen deposition, the clinical results are not convincing [9,10]. Thus, novel approaches to address the disturbed balance in keloid tissue are needed.

Accordingly, it has been an object of the present invention to provide for an improved way of preventing, reducing or treating hypertrophic scars, keloids, or related fibroproliferative disorders.

### Summary of the invention

The objects of the present invention are solved by an S100 family protein for use in the prevention, reduction, or treatment of a hypertrophic scar, keloid, or fibroproliferative disorder, wherein said S100 family protein is selected from S100A7 protein and S100A15 protein, and wherein said protein is administered to a patient in need thereof.

In one embodiment said fibroproliferative disorder is atherosclerosis, hepatic cirrhosis, or pulmonary fibrosis.

In one embodiment said S100A7 protein is a protein having an amino acid sequence of SEQ ID NO:1 or is a protein having an amino acid sequence which is at least 90%, preferably at least 95%, more preferably at least 98%, identical to SEQ ID NO:1.

In one embodiment said S100A15 protein is a protein having an amino acid sequence of SEQ ID NO:2 or is a protein having an amino acid sequence which is at least 90%, preferably at least 95%, more preferably at least 98%, identical to SEQ ID NO:2.

In one embodiment said patient is a mammal, preferably a human being.

In one embodiment said protein is administered intradermally, preferably by injection, and/or topically, or is administered systemically, preferably by injection or ingestion.

In one embodiment said intradermal administration occurs in a dose range of from 1 µg to 50 µg per cm², preferably from 1 µg to 10 µg per cm², of skin surface affected by said disorder, preferably per cm² of skin surface affected by hypertrophic scar formation or keloid formation.

In one embodiment said topical administration occurs in a dose range of from 10 µg to 500 µg per cm², preferably from 10 µg to 100 µg per cm², of skin surface which said S100 family protein is applied to.

In one embodiment said administration occurs once a week, if said protein is administered intradermally, or twice a day, if said protein is administered topically.

In one embodiment said administration occurs before, during, and/or after said patient is inflicted a skin wound.

In one embodiment said protein is used in combination with at least one other, different S100 family protein.

In one embodiment said at least one other, different S100 family protein is selected from the group comprising S100A7 protein and S100A15 protein.

In a preferred embodiment, a combination of S100A7 protein and S100A15 protein is used. In a particularly preferred embodiment, no other S100 family protein is used in conjunction with said combination of S100A7 protein and S100A15 protein.

In one embodiment said administration of said protein to said patient in need thereof is the only preventive or therapeutic treatment in respect of said skin disorder to which said patient is subjected.

In one embodiment simultaneously with said administration of said protein to said patient in need thereof said patient is subjected to one or more additional preventive or therapeutic treatments for treating skin disorders selected from the group comprising pressure therapy, silicone gel therapy, application of flavonoids, preferably of quercetin and/or kaempferol, application of corticosteroids, preferably of a triamcinolone compound, cryotherapy, scar excision, radiotherapy, laser therapy, application of interferon, preferably of interferon-α2b, application of 5-fluorouracil, application of imiquimod, inhibition of TGF-β1 and/or TGF-β2, and application of TGF-β3.

In one embodiment said protein is administered externally.

In one embodiment said protein is generated inside said patient by protein expression from a nucleic acid introduced into said patient.

In one embodiment said protein is non-toxic to human fibroblast cells.

In one embodiment, said protein, when administered to human fibroblast cells in a concentration of 0.01 µg/ml in a cell culture experiment according to Example 2, leads to a decrease in cell vitality which is less than 40%, preferably less than 20%, more preferably less than 10%, when compared to untreated cells.

In one embodiment said protein has therapeutic effects against both hypertrophic scars and keloids.

The objects of the present invention are also solved by a pharmaceutical composition comprising a pharmaceutically acceptable carrier and an S100 family protein as defined above for use in the prevention, reduction or treatment of a hypertrophic scar, keloid, or fibroproliferative disorder, wherein said pharmaceutical composition is administered to a patient in need thereof.

The objects of the present invention are also solved by a method for prevention, reduction, or treatment of a hypertrophic scar, keloid, or fibroproliferative disorder, said method comprising administering an S100 family protein to a patient in need thereof.

In such method, said hypertrophic scar, keloid, fibroproliferative disorder, S100 family protein, and patient are as defined in the embodiments above.

The objects of the present invention are also solved by the use of an S100 family protein in the manufacture of a medicament for the prevention, reduction, or treatment of a hypertrophic scar, keloid, or fibroproliferative disorder.

In one embodiment, said prevention, reduction, or treatment occurs by administration of said S100 family protein to a patient in need thereof.

In such use and the embodiment referring to it, said S100 family protein, hypertrophic scar, keloid, fibroproliferative disorder, and patient are as defined in the embodiments above.

### Detailed description of the invention

The present inventors have surprisingly found that the S100 family proteins S100A7 and S100A15 are highly effective in the treatment of skin disorders that can be alleviated and/or prevented by reduction of collagen synthesis and/or increase of collagen breakdown, such as hypertrophic scars and keloids, as well as fibroproliferative disorders.

Genes of the S100 family encode small (9-13kDa), calcium-binding proteins of the EF-hand type. S100 proteins are effectors of calcium-dependent processes and intervene with the control of cell cycle, cell growth, cell differentiation, as well as chemoattractants for inflammatory cells ([11], reviewed in: [12]). Hence, the expression of S100 proteins is associated with epidermal maturation, inflammation and wound-healing [13-15].

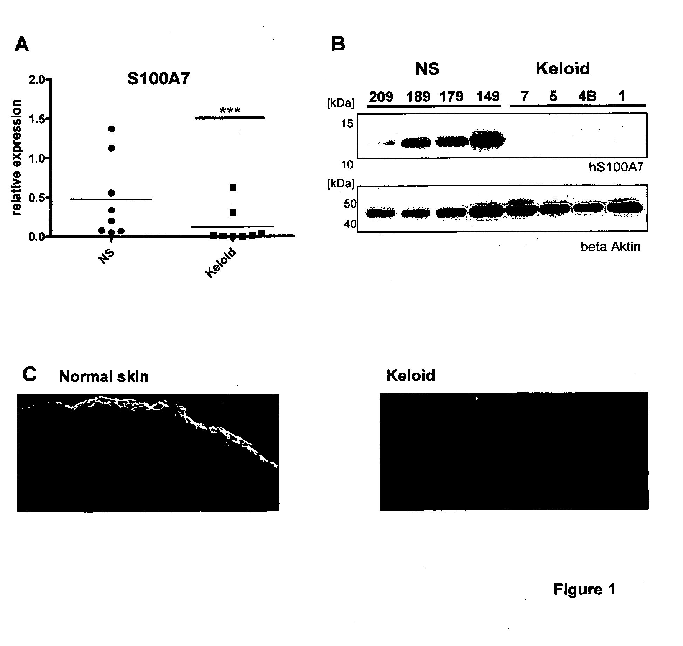

The present inventors have carried out an investigation of the role of S100 proteins in keloid formation. Surprisingly, it was found that S100A7 (Psoriasin) expression and production are significantly decreased in keloid scar tissue compared to normal skin (Fig. 1A, B). Immunostaining of whole tissue biopsies showed that S100A7 expression is markedly downregulated in keloid derived epidermis with S100A7 barely detectable (Fig. 1C). This data set suggests that sub-epidermal fibroblasts in keloid tissue are exposed to decreased levels of secreted S100A7.

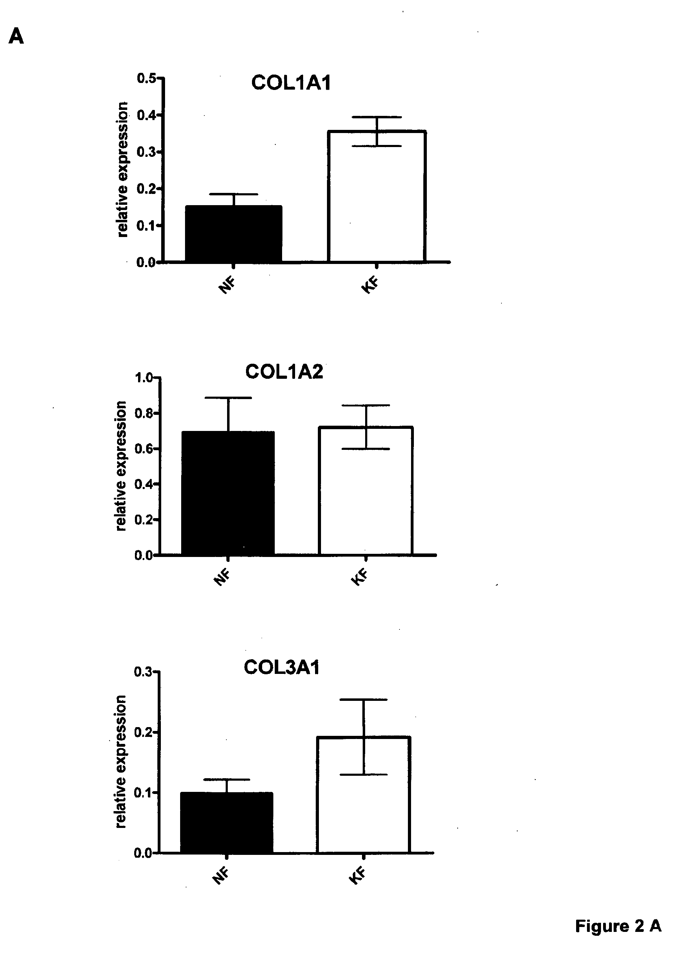

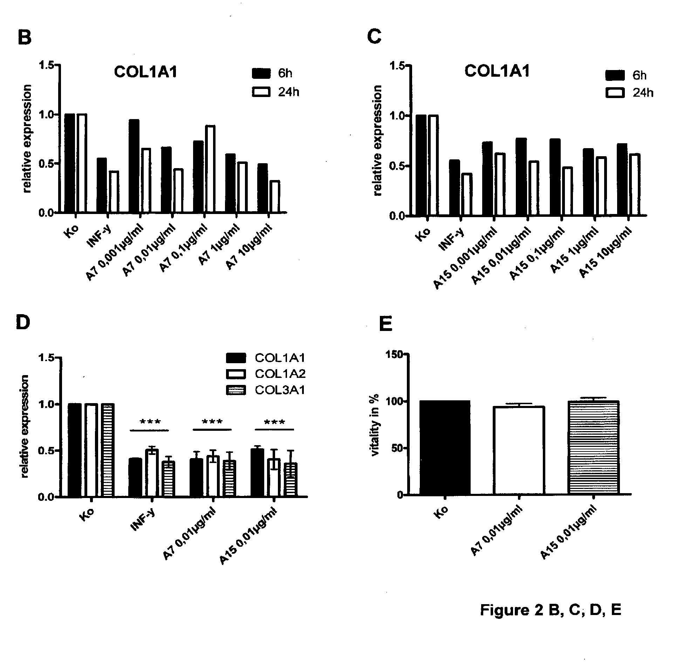

In further experiments, primary fibroblasts from normal skin and keloid tissue were isolated, cultured and collagen expression was determined. Expression of collagens COL1A1, and COL3A1 in keloid derived fibroblasts (KF) was markedly increased (Fig. 2A). To test if S100 proteins affect collagen production, isolated normal fibroblasts (NF) were cultured and stimulated with S100 proteins at different concentrations. Quite surprisingly, data showed that both S100A7 (Psoriasin) and S100A15 (Koebnerisin) inhibited the expression of COL1A1, COL1A2, and COL3A1 (Fig. 2B, C, D). The effects of S100 proteins were similar to interferon-γ, previously established as one of the most potent inhibitors of fibroblast collagen synthesis (Fig. 2D). Cell viability was not affected by either protein (Fig. 2E).

These findings allow the conclusion that the S100 family proteins S100A7 and S100A15 can be used to reduce the amount of collagen produced by skin cells, and thus are effective in the prevention, reduction, or treatment of hypertrophic scars and keloids.

As used herein, the term "S100 family protein" refers to a protein that is a member of the S100 protein family. The term is also meant to encompass proteins with an amino acid sequence at least 90%, preferably 95%, more preferably 98%, identical to the amino acid sequence of a protein that is a member of the S100 protein family. Furthermore, the term is meant to encompass proteins with an amino acid sequence derived by deletion, insertion, or addition of amino acids from (a) the amino acid sequence of a member of the S100 protein family, or (b) from the amino acid sequence of a protein with an amino acid sequence at least 90%, preferably 95%, more preferably 98%, identical to the amino acid sequence of a protein that is a member of the S100 protein family, wherein said protein with an amino acid sequence derived by deletion, insertion, or addition of amino acids is able to cause a reduction of collagen synthesis and/or an increase of collagen breakdown in human fibroblast cells. The S100 protein family is a multigenic family of non-ubiquitous proteins of the EF-hand type that is expressed in vertebrates exclusively. Currently known members of the S100 family include proteins S100A1, S100A2, S100A3, S100A4, S100A5, S100A6, S100A7 (Psoriasin), S100A8, S100A9, S100A10, S100A11, S100A12, S100A13, S100A14, S100A15 (Koebnerisin), S100A16, S100B, S100P, S100Z, CRNN, FLG, HRNR, IFPS, RPTN, S100G, TCHH, and THHL1.

The term "S100A7 protein" is meant to designate a protein with the amino acid sequence given by SEQ ID NO:1, or with an amino acid sequence at least 90%, preferably 95%, more preferably 98%, identical to the amino acid sequence given by SEQ ID NO:1. Furthermore, the term is meant to encompass proteins with an amino acid sequence derived by deletion, insertion, or addition of amino acids from (a) the amino acid sequence given by SEQ ID NO:1, or (b) from an amino acid sequence at least 90%, preferably 95%, more preferably 98%, identical to the amino acid sequence given by SEQ ID NO:1, wherein said protein with an amino acid sequence derived by deletion, insertion, or addition of amino acids is able to cause a reduction of collagen synthesis and/or an increase of collagen breakdown in human fibroblast cells.

The term "S100A15 protein" is meant to designate a protein with the amino acid sequence given by SEQ ID NO:2, or with an amino acid sequence at least 90%, preferably 95%, more preferably 98%, identical to the amino acid sequence given by SEQ ID NO: 2. Furthermore, the term is meant to encompass proteins with an amino acid sequence derived by deletion, insertion, or addition of amino acids from (a) the amino acid sequence given by SEQ ID NO:2, or (b) from an amino acid sequence at least 90%, preferably 95%, more preferably 98%, identical to the amino acid sequence given by SEQ ID NO:2, wherein said protein with an amino acid sequence derived by deletion, insertion, or addition of amino acids is able to cause a reduction of collagen synthesis and/or an increase of collagen breakdown in human fibroblast cells.

"Fibroproliferative disorder", as used herein, refers to a disorder characterized by excessive proliferation of fibroblast cells and/or connective tissue. The term encompasses, but is not limited to pulmonary fibroses, systemic sclerosis, liver cirrhosis, liver fibrosis, cardiovascular idiopathic pulmonary fibrosis, and atherosclerosis.

"Hypertrophic scars" and "keloids" are forms of excessive scar tissue formation. These skin disorders have specific clinical, histological, biochemical, and molecular characteristics known to the person skilled in the art. Notably, while both scar types rise above skin level, only keloids typically project beyond the original wound margins, whereas hypertrophic scars do not extend beyond the initial site of injury. Further differences between these two types of skin disorders are known to the skilled person and documented in detail in the relevant literature (see, e.g., [6]).

At some instances, the present application refers to a percentage to which a first amino acid sequence is "identical" to a second amino acid sequence. This percentage is determined by aligning the two amino acid sequences using appropriate algorithms, which are known to the person skilled in the art, using default parameters; determining the number of identical amino acids in the aligned portion(s); dividing that number by the total number of amino acids in the second amino acid sequence; and then multiplying the resulting number by 100 to obtain the percentage of identical amino acids.

The term "intradermal", as used herein in the context of intradermal administration, refers to an administration that results in the administered substance or substance mixture being located within or between the layers of the skin. This may be achieved for example by injection of the substance or substance mixture between the layers of the skin.

The term "topical", as used herein in the context of topical administration, refers to an administration of a substance or substance mixture that occurs by applying the substance or substance mixture to the surface of the skin, preferably the surface of an area of the skin affected by a skin disorder.

The phrase "is used in combination with", as used herein in the context of a first substance or substance mixture being used in combination with a second substance or substance mixture, is meant to designate that the first substance or substance mixture is administered in conjunction with the second substance or substance mixture. The administration may be simultaneous or one after the other, it may be via the same route or via different routes, it may be in the same dosage form or via separate dosage forms.

The phrase that something/an event occurs "simultaneously with said administration of said protein to said patient in need thereof' is meant to designate that the event occurs (a) at the same time at which said protein is applied to the patient, (b) at a time that lies between individual administrations of said protein, if administration of said protein occurs in several individual administrations, or (c) after administration of said protein, while the administered protein is still present in the tissue, blood, or digestive system of the patient or on the surface of the skin of the patient.

At some instances, the present application refers to a number of preventive and/or therapeutic treatments for skin disorders, such as pressure therapy, silicone gel therapy, application of flavonoids, application of corticosteroids, cryotherapy, scar excision, radiotherapy, laser therapy, application of interferon, application of 5-fluorouracil, application of imiquimod, inhibition of TGF-β1 and/or TGF-β2, and application of TGF-β3. These treatments are known to the skilled person. They are referred to, for example, in reference [1] and described in detail in the literature of the field.

When the present application refers to a protein being "generated inside said patient by protein expression from a nucleic acid introduced into said patient ", this means that a nucleic acid, such as a DNA, is introduced into a cell of said patient, for example a skin cell, for example by means of a viral vector carrying said sequence. By protein expression from said nucleic acid, a protein with the amino acid sequence encoded by said nucleic acid is generated inside said patient.

The term "administered externally", as used herein in the context of a protein being administered externally, refers to a way of delivering a protein in which the protein is not delivered by generating it inside said patient by protein expression, but in which the protein is delivered from the outside, optionally in the form of a precursor protein.

The term "pharmaceutically acceptable carrier" refers to a non-toxic, inert, solid, semi-solid, or liquid diluent material, or formulation auxiliary of any type. "Pharmaceutically acceptable" in this context is meant to designate that said carrier is compatible with the other ingredients of the pharmaceutical composition and not harmful to the patient that the pharmaceutical composition is administered to. Examples of pharmaceutically acceptable carriers include, but are not limited to, water, water-propylene glycol solutions, or aqueous polyethylene glycol solutions.

### Figure legends

Figure 1
   Figure 1 shows decreased S100A7 expression and production in keloid tissue.
   Panel A shows the result of real-time qPCR (real-time quantitative Polymerase Chain Reaction) for S100A7 expression carried out from RNA of normal skin (NS) and keloid scar tissue prepared according to Example 1.
   Panel B shows Western blot analysis of whole cell lysates probed with specific antibodies against S100A7 and β-actin (loading control) according to Example 1. hS100A7: anti-human S100A7. beta Aktin: anti-human-β-Actin
   Panel C shows immunostaining for S100A7 in frozen skin tissue sections from normal subjects (left) and patients with keloids (right). Scale bar: 20 µm. While in skin sections from normal subjects a bright immunostaining signal is observed in the epidermis, no such signal is observed in the epidermis of keloid skin sections, thus demonstrating marked downregulation of S100A7 in the epidermis of keloids.
Figure 2
   Figure 2 shows inhibition of COL1A1, COL1A2, and COL3A1 expression in human fibroblasts by S100A7 and S100A15.
   Panel A shows the result of real-time qPCR for COL1A1, COL1A2, and COL3A1 from total RNA of normal fibroblasts (NF) and keloid fibroblasts (KF) according to Example 2. Expression of COL1A1 and COL3A1 in keloid derived fibroblasts (KF) is increased compared to normal fibroblasts (NF).
   Panel B shows the result of real-time qPCR for COL1A1 from total RNA extracted after 6 hours and 24 hours from NF cells treated with the indicated concentrations of S100A7 or interferon-γ according to Example 2. COL1A1 expression in human fibroblasts is inhibited by S100A7.
   Panel C shows the result of real-time qPCR for COL1A1 from total RNA extracted after 6 hours and 24 hours from NF cells treated with the indicated concentrations of S100A15 or interferon-γ ("INF-y") according to Example 2. COL1A1 expression in human fibroblasts is inhibited by S100A15.
   Panel D shows the result of real-time qPCR for COL1A1, COL1A2, and COL3A1 from total RNA extracted after 24 hours from NF cells treated with the indicated concentrations of S100A7 or S100A15, respectively, according to Example 2. COL1A1 expression in human fibroblasts is inhibited by S100A7 and S100A15.
   Panel E shows the number of viable cells in cultured fibroblasts treated with S100A7 and S100A15 and stained with crystal violet according to Example 2.
   NF: normal fibroblasts. KF: keloid derived fibroblasts. Ko: control. INF-y: interferon-γ treatment. A7: S100A7 treatment with the indicated concentrations. A15: S100A15 treatment with the indicated concentrations.
Figure 3

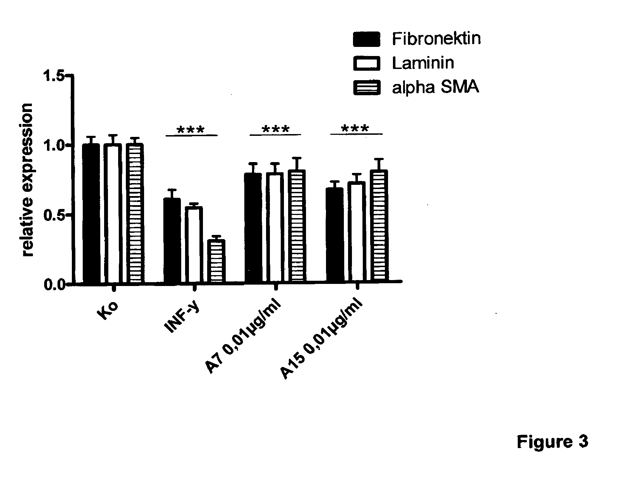

   Figure 3 shows inhibition of fibronectin, laminin and alpha smooth muscle actin expression in human fibroblasts by treatment with S100A7 or S100A15, as measured by real-time qPCR.
   Ko: control. INF-y: interferon-γ treatment (0.1 µg/ml). A7: S100A7 treatment (0.01 µg/ml). A15: S100A15 treatment (0.01 µg/ml).
Figure 4

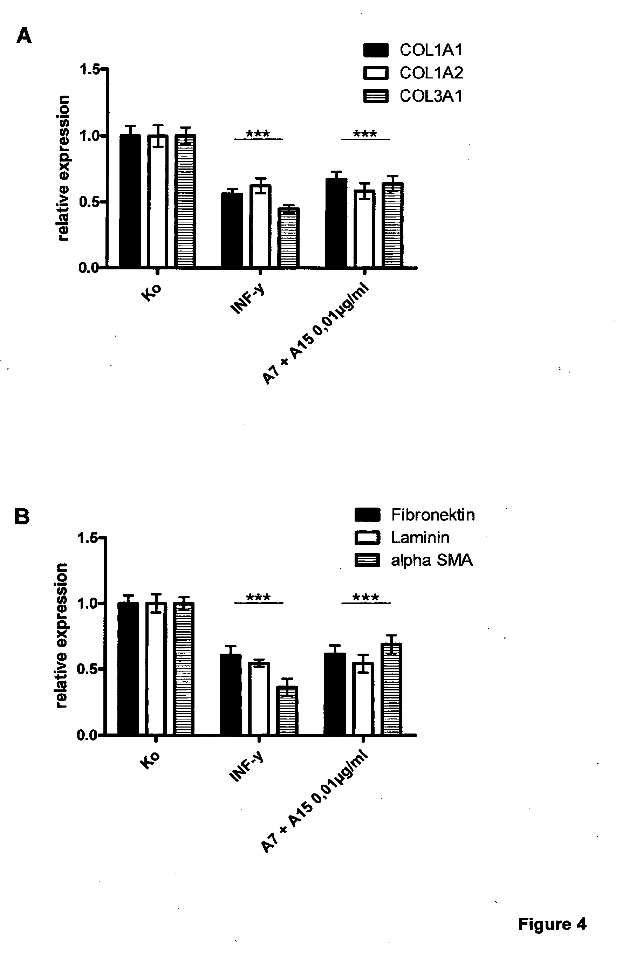

   Figure 4 shows measurements by real-time qPCR demonstrating inhibition of COL1A1, COL1A2, and COL3A1 (Panel A), fibronectin, laminin and alpha smooth muscle actin (Panel B) expression in human fibroblasts upon treatment with a combination of S100A7 and S100A15 (0.01 µg/ml each), in comparison to treatment with interferon-γ (0.1 µg/ml).
   Ko: control. INF-y: interferon-γ treatment (0.1 µg/ml). A7 + A15: treatment with a combination of S100A7 (0.01 µg/ml) and S100A15 (0.01 µg/ml).
Figure 5

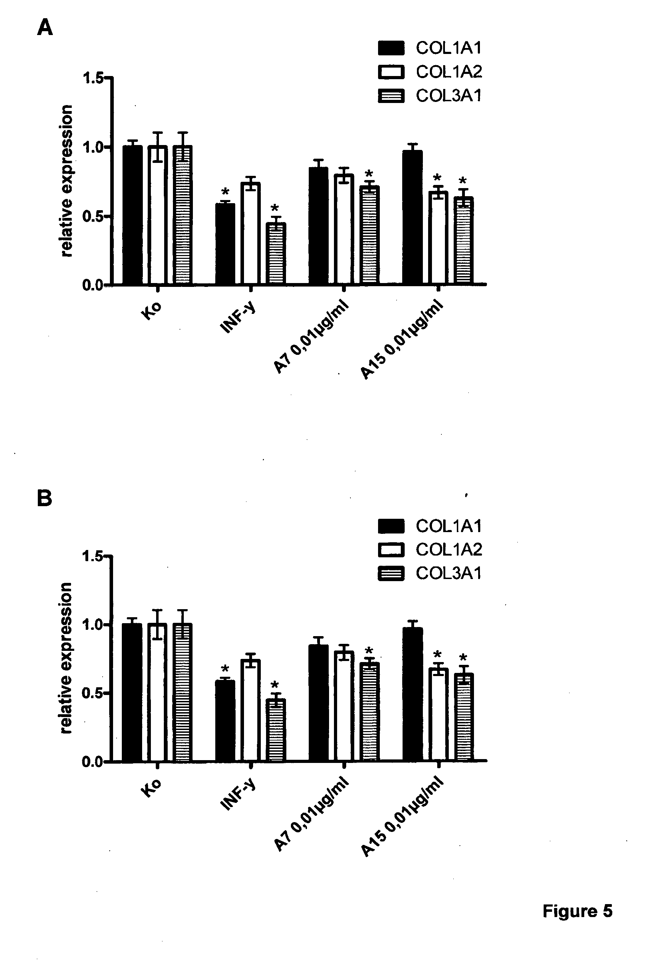

   Figure 5 demonstrates inhibition of COL1A1, COL1A2, and COL3A1 expression in human keloid-derived fibroblasts by either S100A7 (0.01 µg/ml), or S100A15 (0.01 µg/ml), in comparison to the inhibition by interferon-γ (0.1 µg/ml). Measurements were carried out by real-time qPCR.
   Ko: control. INF-y: interferon-γ treatment (0.1 µg/ml). A7: S100A7 treatment (0.01 µg/ml). A15: S100A15 treatment (0.01 µg/ml).
Figure 6
   Figure 5 shows inhibition of fibroblast proliferation upon treatment with a combination of S100A7 (0.01 µg/ml) and S100A15 (0.01 µg/ml), as determined by BrdU Assay.
   NK: normal control. INF-y: interferon-γ treatment (0.1 µg/ml). S100A7: S100A7 treatment (0.01 µg/ml). S100A15: S100A15 treatment (0.01 µg/ml).

### Examples

The invention will now be further described by the following examples, which are given to illustrate the invention, not to limit it.

### Example 1

Primary normal and keloid fibroblasts were isolated from normal skin tissue and keloid scar tissue. Tissue samples were cut into small pieces and placed on the plate, with the dermis on top. The samples were incubated between ten and twenty days in Dulbecco's modified Eagle's medium (DMEM) supplemented with 10% fetal bovine serum (FBS), ampicillin/streptomycin and amphotericin at 37 °C in a 5% CO₂ atmosphere. Fibroblasts grew out of the tissue and were then trypsinized. Only cells from passage 3 - 5 were used for the experiments. Cells were seeded into 12 well or 6 well plates in DMEM with 10% FBS. After three days, medium was changed into serum-free medium for 24 h. Then the cells were stimulated with S100A7, S100A15 or interferon-γ in serum-free DMEM for further 6 h or 24 h.

Total RNA of normal skin (n=8) and keloid scar tissue (n=8) was extracted, and real-time qPCR for S100A7 expression was performed (see Figure 1, Panel A).

Whole cell lysates (15 µg per lane) were probed with specific antibodies against hS100A7 and β-actin in Western blots (see Figure 1, Panel B).

Immunostaining for hS100A7 was carried out in frozen skin tissue sections from normal subjects and patients with keloid (see Figure 1, Panel C).

### Example 2

Total RNA of normal fibroblasts (n=3) and keloid fibroblasts (n=3) was extracted, and real-time qPCR was performed (see Figure 2, Panel A).

NF (normal fibroblast) cells were either treated with various concentrations of S100A7 or S100A15 in the culture media (0.001 µg/ml, 0.01 µg/ml, 0.1 µg/ml, 1 µg/ml, or 10 µg/ml). After six and 24 hours, total RNA was extracted from the cells, and real-time qPCR was performed (see Figure 2, Panels B, C, D).

Cultured fibroblasts treated with S100A7 or S100A15 were stained with crystal violet and numbers of viable cells were measured (see Figure 2, Panel E).

### Example 3

The effect of S100A7 and S100A15 on fibroblast proliferation was determined by BrdU Assay (Roche Applied Science, Mannheim, Germany) according to manufacturer's instructions. Cultured fibroblasts were exposed to INF-y (0.1 µg/ml), and to S100A15 and S100A7 (each 0.01µg/ml) in combination for 48 hours. At 24 hours, 5-bromo-2'-deoxyuridine (BrdU) labelling medium was added, and incoporated BrdU during cell proliferation was detected by an anti-BrdU HRP-labelled antibody. Absorbance was measured at 570 nm using a microplate reader.

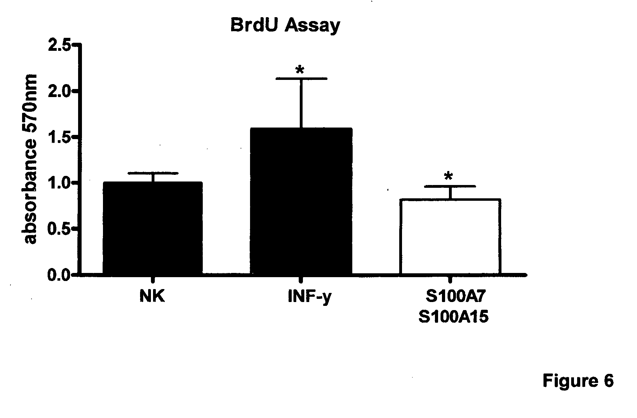

Incubation of cultured fibroblasts with collagen-effective concentrations of combined S100A7 and S100A15 resulted in significantly decreased absorbance compared to untreated fibroblasts indicating a marked decrease in fibroblast proliferation. Incubation with INF-y did not decrease fibroblast proliferation, in contrast, INF-y treatment led to solid increases of fibroblast proliferation as shown in Figure 6.

The features of the present invention disclosed in the specification, the claims, and/or in the accompanying drawings may, both separately and in any combination thereof, be material for realizing the invention in various forms thereof.

### References

1. Marneros, A.G. and T. Krieg, Keloids--clinical diagnosis, pathogenesis, and treatment options. J Dtsch Dermatol Ges, 2004. 2(11): p. 905-13.
2. Ladak, A. and E.E. Tredget, Pathophysiology and management of the burn scar. Clin Plast Surg, 2009. 36(4): p. 661-74.
3. Brown, J.J. and A. Bayat, Genetic susceptibility to raised dermal scarring. Br J Dermatol, 2009. 161(1): p. 8-18.
4. Armour, A., P.G. Scott, and E.E. Tredget, Cellular and molecular pathology of HTS: basis for treatment. Wound Repair Regen, 2007. 15 Suppl 1: p. S6-17.
5. Butler, P.D., M.T. Longaker, and G.P. Yang, Current progress in keloid research and treatment. J Am Coll Surg, 2008. 206(4): p. 731-41.
6. Köse, O. and A. Waseem, Keloids and hypertrophic scars: are they two different sides of the same coin? Dermatol Surg, 2008. 34(3): p. 336-46.
7. Szulgit, G., et al., Alterations in fibroblast alpha1beta1 integrin collagen receptor expression in keloids and hypertrophic scars. J Invest Dermatol, 2002. 118(3): p. 409-15.
8. Bock, O., et al., Studies of transforming growth factors beta 1-3 and their receptors I and II in fibroblast of keloids and hypertrophic scars. Acta Derm Venereol, 2005. 85(3): p. 216-20.
9. Ferguson, M.W., et al., Prophylactic administration of avotermin for improvement of skin scarring: three double-blind, placebo-controlled, phase I/II studies. Lancet, 2009. 373(9671): p. 1264-74.
10. Shah, M., D.M. Foreman, and M.W. Ferguson, Neutralisation of TGF-beta 1 and TGF-beta 2 or exogenous addition of TGF-beta 3 to cutaneous rat wounds reduces scarring. J Cell Sci, 1995. 108 (Pt 3): p. 985-1002.
11. Berridge, M.J., M.D. Bootman, and H.L. Roderick, Calcium signalling: dynamics, homeostasis and remodelling. Nat Rev Mol Cell Biol, 2003. 4(7): p. 517-29.
12. Donato, R., S100: a multigenic family of calcium-modulated proteins of the EF-hand type with intracellular and extracellular functional roles. Int J Biochem Cell Biol, 2001. 33(7): p. 637-68.
13. Schafer, B.W. and C.W. Heizmann, The S100 family of EF-hand calcium-binding proteins: functions and pathology. Trends Biochem Sci, 1996. 21(4): p. 134-40.
14. Watson, P.H., E.R. Leygue, and L.C. Murphy, Psoriasin (S100A7). Int J Biochem Cell Biol, 1998. 30(5): p. 567-71.
15. Kim, E.J. and D.M. Helfman, Characterization of the metastasis-associated protein, S100A4. Roles of calcium binding and dimerization in cellular localization and interaction with myosin. J Biol Chem, 2003. 278(32): p. 30063-73.

### SEQUENCES

SEQ ID NO:1 is the protein sequence of human S100A7 (hS100A7), SEQ ID NO:2 is the protein sequence of human S100A15 (hS100A15).
SEQ ID NO:1
SEQ ID NO:2

## Claims

1. S100 family protein for use in the prevention, reduction, or treatment of a hypertrophic scar, keloid, or fibroproliferative disorder,
wherein said S100 family protein is selected from S100A7 protein and S100A15 protein,
and wherein said protein is administered to a patient in need thereof.

2. S100 family protein for use according to claim 1, wherein said fibroproliferative disorder is atherosclerosis, hepatic cirrhosis, or pulmonary fibrosis.

3. S100 family protein for use according to claim 1 or 2, wherein said S100A7 protein is a protein having an amino acid sequence of SEQ ID NO:1 or is a protein having an amino acid sequence which is at least 90%, preferably at least 95%, more preferably at least 98%, identical to SEQ ID NO:1, and wherein said S100A15 protein is a protein having an amino acid sequence of SEQ ID NO:2 or is a protein having an amino acid sequence which is at least 90%, preferably at least 95%, more preferably at least 98%, identical to SEQ ID NO:2.

4. S100 family protein for use according to any of the preceding claims, wherein said patient is a mammal, preferably a human being.

5. S100 family protein for use according to any of the preceding claims, wherein said protein is administered intradermally, preferably by injection, and/or topically, or is administered systemically, preferably by injection or ingestion.

6. S100 family protein for use according to claim 5, wherein said intradermal administration occurs in a dose range of from 1 µg to 50 µg per cm², preferably from 1 µg to 10 µg per cm², of skin surface affected by said disorder, preferably per cm² of skin surface affected by hypertrophic scar formation or keloid formation.

7. S100 family protein for use according to claim 5, wherein said topical administration occurs in a dose range of from 10 µg to 500 µg per cm², preferably from 10 µg to 100 µg per cm², of skin surface which said S100 family protein is applied to.

8. S100 family protein for use according to any of the preceding claims, wherein said administration occurs once a week, if the said protein is administered intradermally, or twice a day, if said protein is administered topically.

9. S100 family protein for use according to any of the preceding claims, wherein said administration occurs before, during, and/or after said patient is inflicted a skin wound.

10. S100 family protein for use according to any of the preceding claims, wherein said protein is used in combination with at least one other, different S100 family protein.

11. S100 family protein for use according to claim 10, wherein said at least one other, different S100 family protein is selected from the group comprising S100A7 protein and S100A15 protein.

12. S100 family protein for use according to any of the preceding claims, wherein said administration of said protein to said patient in need thereof is the only preventive or therapeutic treatment in respect of said skin disorder to which said patient is subjected, or wherein simultaneously with said administration of said protein to said patient in need thereof said patient is subjected to one or more additional preventive or therapeutic treatments for treating skin disorders selected from the group comprising pressure therapy, silicone gel therapy, application of flavorloids, preferably of quercetin and/or kaempferol, application of corticosteroids, preferably of a triamcinolone compound, cryotherapy, scar excision, radiotherapy, laser therapy, application of interferon, preferably of interferon-α2b, application of 5-fluorouracil, application of imiquimod, inhibition of TGF-β1 and/or TGF-β2, and application of TGF-β3.

13. S100 family protein for use according to any of the preceding claims, wherein said protein is administered externally.

14. S100 family protein for use according to any of claims 1 to 5 or 9 to 13, wherein said protein is generated inside said patient by protein expression from a nucleic acid introduced into said patient.

15. Pharmaceutical composition comprising a pharmaceutically acceptable carrier and an S100 family protein as defined in any of claims 1 to 14 for use in the prevention, reduction or treatment of hypertrophic scars, keloids, or a fibroproliferative disorder,
wherein said pharmaceutical composition is administered to a patient in need thereof.

## Patentansprüche

1. S100-Familie-Protein zur Verwendung bei der Verhinderung, Verringerung oder Behandlung einer hypertrophen Narbe, Keloid oder fibroproliferativen Erkrankung, wobei das S100-Familie-Protein ausgewählt ist aus S100A7-Protein und S100A15-Protein, und wobei das Protein an einen hierfür bedürftigen Patienten verabreicht wird.

2. S100-Familie-Protein zur Verwendung nach Anspruch 1, wobei die fibroproliferative Erkrankung Arteriosklerose, Leberzirrhose oder Lungenfibrose ist.

3. S100-Familie-Protein zur Verwendung nach Anspruch 1 oder 2, wobei das S100A7-Protein ein Protein mit einer Aminosäuresequenz von SEQ ID NO: 1 ist oder ein Protein mit einer Aminosäuresequenz ist, die wenigstens 90%, bevorzugt wenigstens 95%, bevorzugter wenigstens 98% mit SEQ ID NO: 1 identisch ist, und wobei das S100A15-Protein ein Protein mit einer Aminosäuresequenz von SEQ ID NO: 2 ist oder ein Protein mit einer Aminosäuresequenz ist, die wenigstens 90%, bevorzugt wenigstens 95%, bevorzugter wenigstens 98% mit SEQ ID NO: 2 identisch ist.

4. S100-Familie-Protein zur Verwendung nach einem der vorangehenden Ansprüche, wobei der Patient ein Säugetier ist, bevorzugt ein Mensch.

5. S100-Familie-Protein zur Verwendung nach einem der vorangehenden Ansprüche, wobei das Protein intradermal verabreicht wird, bevorzugt mittels Injektion, und/oder topisch, oder systemisch verabreicht wird, bevorzugt mittels Injektion oder Ingestion.

6. S100-Familie-Protein zur Verwendung nach Anspruch 5, wobei die intradermale Verabreichung in einem Dosisbereich von 1 µg bis 50 µg pro cm² stattfindet, bevorzugt von 1 µg bis 10 µg pro cm² der Hautoberfläche, die durch die Erkrankung betroffen ist, bevorzugt pro cm² der Hautoberfläche, die durch hypertrophe Narbenbildung oder Keloidbilddung betroffen ist.

7. S100-Familie-Protein zur Verwendung nach Anspruch 5, wobei die topische Verabreichung in einem Dosisbereich von 10 µg bis 500 µg pro cm², bevorzugt von 10 µg bis 100 µg pro cm² der Hautoberfläche stattfindet, auf die das S100-Familie-Protein aufgetragen wird.

8. S100-Familie-Protein zur Verwendung nach einem der vorangehenden Ansprüche, wobei die Verabreichung einmal pro Woche stattfindet, wenn das Protein intradermal verabreicht wird, oder zweimal am Tag, wenn das Protein topisch verabreicht wird.

9. S100-Familie-Protein zur Verwendung nach einem der vorangehenden Ansprüche, wobei die Verabreichung stattfindet, bevor, während und/oder nachdem dem Patienten eine Hautwunde beigebracht worden ist.

10. S100-Familie-Protein zur Verwendung nach einem der vorangehenden Ansprüche, wobei das Protein in Kombination mit wenigstens einem anderen unterschiedlichen S100-Familie-Protein verwendet wird.

11. S100-Familie-Protein zur Verwendung nach Anspruch 10, wobei das wenigstens eine andere unterschiedliche S100-Familie-Protein ausgewählt ist aus der Gruppe, umfassend S100A7-Protein und S100A15-Protein.

12. S100-Familie-Protein zur Verwendung nach einem der vorangehenden Ansprüche, wobei die Verabreichung des Proteins an den hierfür bedürftigen Patienten die einzige präventive oder therapeutische Behandlung bezüglich der Hauterkrankung ist, der der Patient unterworfen wird, oder wobei zeitgleich mit der Verabreichung des Proteins an den hierfür bedürftigen Patienten der Patient einer oder mehreren zusätzlichen präventiven oder therapeutischen Behandlungen zum Behandeln von Hauterkrankungen unterzogen wird, ausgewählt aus der Gruppe, umfassend Drucktherapie, Silikongeltherapie, Verabreichung von Flavonoiden, bevorzugt von Quercetin und/oder Kaempferol, Verabreichung von Kortikosteroiden, bevorzugt einer Triamcinolonverbindung, Cryotherapie, Narbenausschneidung, Strahlungstherapie, Lasertherapie, Verabreichung von Interferon, bevorzugt von Interferon-α2b, Verabreichung von 5-Fluorouracil, Verabreichung von Imiquimod, Inhibition von TGF-β1 und/oder TGF-β2, und Verabreichung von TGF-β3.

13. S100-Familie-Protein zur Verwendung nach einem der vorangehenden Ansprüche, wobei das Protein extern verabreicht wird.

14. S100-Familie-Protein zur Verwendung nach einem der Ansprüche 1-5 oder 9-13, wobei das Protein innerhalb des Patienten mittels Proteinexpression von einer Nukleinsäure erzeugt wird, die in den Patienten eingeführt worden ist.

15. Pharmazeutische Zusammensetzung, umfassend einen pharmazeutisch akzeptablen Träger und ein S100-Familie-Protein, wie in einem der Ansprüche 1-14 definiert, zur Verwendung bei der Prävention, Verringerung oder Behandlung von hypertrophen Narben, Keloiden oder einer fibroproliferativen Erkrankung, wobei die pharmazeutische Zusammensetzung an einen hierfür bedürftigen Patienten verabreicht wird.

## Revendications

1. Protéine de la famille S100, pour son utilisation dans la prévention, la réduction ou le traitement d'une cicatrice hypertrophique, d'une chéloïde ou d'un trouble fibroprolifératif,
dans laquelle ladite protéine de la famille S100 est choisie parmi la protéine S100A7 et la protéine S100A15,
et dans laquelle ladite protéine est administrée à un patient en ayant besoin.

2. Protéine de la famille S100 pour son utilisation selon la revendication 1, dans laquelle ledit trouble fibroprolifératif est l'athérosclérose, la cirrhose hépatique ou la fibrose pulmonaire.

3. Protéine de la famille S100 pour son utilisation selon la revendication 1 ou 2, dans laquelle ladite protéine S100A7 est une protéine possédant une séquence d'acides aminés de SEQ ID NO : 1 ou est une protéine possédant une séquence d'acides aminés qui est identique à au moins 90 %, de préférence à au moins 95 %, et de manière davantage préférée à au moins 98 % à SEQ ID NO : 1, et dans laquelle ladite protéine S100A15 est une protéine possédant une séquence d'acides aminés de SEQ ID NO : 2 ou est une protéine possédant une séquence d'acides aminés qui est identique à au moins 90 %, de préférence à au moins 95 %, et de manière davantage préférée à au moins 98 % à SEQ ID NO : 2.

4. Protéine de la famille S100 pour son utilisation selon l'une quelconque des revendications précédentes, dans laquelle ledit patient est un mammifère, de préférence un être humain.

5. Protéine de la famille S100 pour son utilisation selon l'une quelconque des revendications précédentes, dans laquelle ladite protéine est administrée par voie intradermique, de préférence par injection, et/ou par voie topique, ou est administrée par voie systémique, de préférence par injection ou ingestion.

6. Protéine de la famille S100 pour son utilisation selon la revendication 5, dans laquelle ladite administration intradermique est réalisée dans une plage de doses allant de 1 µg à 50 µg par cm², de préférence de 1 µg à 10 µg par cm² de surface cutanée affectée par ledit trouble, de préférence par cm² de surface cutanée affectée par la formation d'une cicatrice hypertrophique ou la formation d'une chéloïde.

7. Protéine de la famille S100 pour son utilisation selon la revendication 5, dans laquelle ladite administration topique est réalisée dans une plage de doses allant de 10 µg à 500 µg par cm², de préférence de 10 µg à 100 µg par cm² de surface cutanée sur laquelle ladite protéine de la famille S100 est appliquée.

8. Protéine de la famille S100 pour son utilisation selon l'une quelconque des revendications précédentes, dans laquelle ladite administration est réalisée une fois par semaine, si ladite protéine est administrée par voie intradermique, ou deux fois par jour si ladite protéine est administrée par voie topique.

9. Protéine de la famille S100 pour son utilisation selon l'une quelconque des revendications précédentes, dans laquelle ladite administration est réalisée avant, pendant et/ou après l'apparition d'une blessure cutanée chez ledit patient.

10. Protéine de la famille S100 pour son utilisation selon l'une quelconque des revendications précédentes, dans laquelle ladite protéine est utilisée en combinaison avec au moins une autre protéine de la famille S100 différente.

11. Protéine de la famille S100 pour son utilisation selon la revendication 10, dans laquelle ladite au moins une autre protéine de la famille S100 différente est choisie dans le groupe comprenant la protéine S100A7 et la protéine S100A15.

12. Protéine de la famille S100 pour son utilisation selon l'une quelconque des revendications précédentes, dans laquelle ladite administration de ladite protéine audit patient en ayant besoin est le seul traitement préventif ou thérapeutique en ce qui concerne ledit trouble cutané auquel ledit patient est soumis, ou dans laquelle simultanément avec ladite administration de ladite protéine audit patient en ayant besoin, ledit patient est soumis à un ou plusieurs traitements préventifs ou thérapeutiques supplémentaires pour traiter des troubles cutanés choisis dans le groupe comprenant une pressothérapie, une thérapie au moyen d'un gel de silicone, l'application de flavonoïdes, de préférence de quercétine et/ou de kaempférol, l'application de corticostéroïdes, de préférence d'un composé de triamcinolone, une cryothérapie, une excision de cicatrice, une radiothérapie, un traitement au laser, l'application d'un interféron, de préférence de l'interféron-α2b, l'application de 5-fluorouracile, l'application d'imiquimod, l'inhibition du TGF-β1 et/ou du TGF-β2, et l'application de TGF-β3.

13. Protéine de la famille S100 pour son utilisation selon l'une quelconque des revendications précédentes, dans laquelle ladite protéine est administrée de manière externe.

14. Protéine de la famille S100 pour son utilisation selon l'une quelconque des revendications 1 à 5 ou 9 à 13, dans laquelle ladite protéine est générée à l'intérieur dudit patient par l'expression de protéines à partir d'un acide nucléique introduit dans ledit patient.

15. Composition pharmaceutique comprenant un support pharmaceutiquement acceptable et une protéine de la famille S100 telle que définie dans l'une quelconque des revendications 1 à 14, pour son utilisation dans la prévention, la réduction ou le traitement des cicatrices hypertrophiques, des chéloïdes ou d'un trouble fibroprolifératif,
et dans laquelle ladite composition pharmaceutique est administrée à un patient en ayant besoin.
